# EUROPEAN PATENT APPLICATION

(11) **EP 2 711 352 A1**
(43) Date of publication of application: **26.03.2014**
(21) Application number: 12784963.6
(22) Date of filing: 18.05.2012
(51) Int. Cl.: C07C 67/343, C07C 17/18, C07C 22/08, C07C 69/618, C07C 69/73, C07F 7/08

(54) **POLYVALENT VINYL AROMATIC COMPOUND AND METHOD FOR PRODUCING SAME**

(30) Priority: 19.05.2011 US 201161487826 P
(71) Applicant: UBE Industries, Ltd., Yamaguchi 755-8633 (JP); Tokyo Institute of Technology, Tokyo 152-8550 (JP)
(72) Inventor: MIKAMI, Koichi, Tokyo 152-8550 (JP); SERIZAWA, Hiroki, Tokyo 152-8550 (JP); SAITO, Norimichi, New York New York 10016 (US)
(74) Representative: Webster, Jeremy Mark
(86) International application number: PCT/JP2012/062833
(87) International publication number: WO 2012/157760

(57) **Abstract**

A polyvalent vinyl aromatic compound represented by the following chemical formula 4 is produced by reacting (1) a specific vinyl aromatic compound and (2) a specific vinyl compound in the presence of (3-1) a metal complex and (3-2) a compound in the class of percarboxylic acids or a hypervalent iodine compound so that a vinyl group derived from the vinyl compound is introduced onto a carbon atom that is adjacent to the carbon atom to which a vinyl group is bonded in the vinyl aromatic compound: 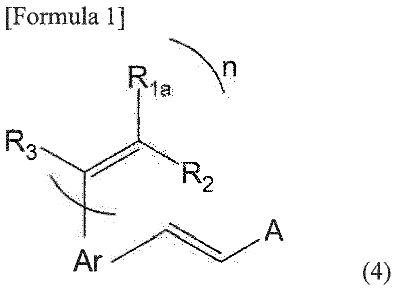 wherein Ar is a monocyclic aromatic hydrocarbon group or a ring-cumulated hydrocarbon group formed by the linkage of a plurality of the aromatic hydrocarbon groups via a single bond or an unsaturated bond, has a vinyl group on a carbon atom, and may have a substituent on a carbon atom that is not adjacent to the carbon atom having the vinyl group; R₁ₐ is a group represented by -CH₂OC(=O)R₄ (wherein R₄ is an alkyl group having 1 to 20 carbon atoms), an alkyl group having 1 to 20 carbon atoms, either a monocyclic aromatic hydrocarbon group which may have a substituent or a ring-cumulated hydrocarbon group which may have a substituent and which is formed by the linkage of a plurality of the aromatic hydrocarbon groups via a single bond or an unsaturated bond, a trialkylsilyl group, a hydrogen atom, or -CH₂OC(=O)R₆ wherein R₆ is an alkyl group having 1 to 5 carbon atoms, a phenyl group, or a phenyl group having, as a substituent, an alkyl group having 1 to 5 carbon atoms; R₂ is a hydrogen atom or an alkyl group having 1 to 5 carbon atoms; R₃ is a hydrogen atom or an alkyl group having 1 to 20 carbon atoms; and n is an integer of 1 to m, provided that when Ar is a monocyclic aromatic hydrocarbon group, m is a number obtained by subtracting 1 from the number of the carbon atoms that constitute the aromatic ring represented by Ar or, when Ar is a ring-cumulated hydrocarbon group, m is a number obtained by subtracting 1 from the number of the carbon atoms that are not involved in a single bond or an unsaturated bond among the carbon atoms that constitute the aromatic ring.

## Description

### TECHNICAL FIELD

The present invention relates to a polyvalent vinyl aromatic compound and to a method for producing the same.

### BACKGROUND ART

Substituted aromatic compounds obtained by introduction of a substituent onto aromatic compounds such as benzene are attracting attention as luminescent materials or liquid crystal materials in the field of displays. To obtain a desired substituted aromatic compound, it is necessary to control the position at which a substituent is introduced. As methods for controlling the position of a substituent, Non-patent Documents 1 to 5 propose methods by which Pd(OAc)₂ is attached to the ortho position of benzene having, as a substituent, a polar group such as a carbonyl group.

For example, Non-patent Documents 1 to 3 disclose methods for attaching Pd(OAc)₂ to the ortho position of benzene having, as a substituent, an acetamide group (-NH-C(=O)-CH₃), a carboxyl group, and an aldehyde group, respectively. Non-patent Document 4 discloses a method for attaching Pd(OAc)₂ to the ortho position of benzene having a 2-methyl-2-hydroxypropyl group (-CH₂C(CH₃)(CH₃)OH) as a substituent, and Non-patent Document 5 discloses a method for attaching Pd(OAc)₂ to the ortho position of benzene having a pyridyl group as a substituent.

### CITATION LIST

### NON-PATENT DOCUMENTS

Non-patent Document 1: de Vries et al., J. Am. Chem. Soc., 124, 1586 (2002)
Non-patent Document 2: Yu et al., J. Am. Chem. Soc., 130, 17676 (2008)
Non-patent Document 3: Yu et al., J. Am. Chem. Soc., 132, 5916 (2010)
Non-patent Document 4: Glorius et al., J. Am. Chem. Soc., 132, 5970 (2010)
Non-patent Document 5: Yu et al., J. Am. Chem. Soc., 128, 12634 (2006)

### SUMMARY OF INVENTION

### TECHNICAL PROBLEM

As a result of preliminary studies on the methods disclosed in Non-patent Documents 1 to 5, the inventors of the present invention found that it is difficult to control the position at which Pd(OAc)₂ is introduced.

In particular, derivatives having two or more unsaturated hydrocarbon groups (for example, vinyl groups) on adjacent carbon atoms (or at the ortho positions of benzene) are conjugated systems and hence, are expected to be applied as luminescent materials having high luminescence intensity or materials for dye-sensitized solar cells. Meanwhile, Non-patent Documents 1 to 5 do not refer to use of unsaturated hydrocarbon groups as substituents.

As mentioned above, a simple method for producing an aromatic compound having two or more vinyl groups on adjacent carbon atoms has been desired, but there has yet to be a satisfactory production method. In light of the circumstances mentioned above, the present invention aims to provide a polyvalent vinyl aromatic compound having two or more vinyl groups on adjacent carbon atoms and a method for producing the compound.

### SOLUTION TO PROBLEM

As a result of studies, the inventors of the present invention found that the object mentioned above can be achieved using a specific metal catalyst. More specifically, the object is achieved by the following inventions.
[1]
   (1) A method for producing a polyvalent vinyl aromatic compound comprising a step of allowing a vinyl aromatic compound represented by the following chemical formula 1: 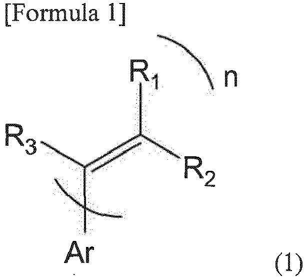
      wherein Ar is a monocyclic aromatic hydrocarbon group or a ring-cumulated hydrocarbon group formed by linkage of a plurality of the aromatic hydrocarbon groups via a single bond or an unsaturated bond, has a vinyl group on a carbon atom, and may have a substituent on a carbon atom that is not adjacent to the carbon atom having the vinyl group,
      R₁ is a group represented by -CH₂OC(=O)R₄ (wherein R₄ is an alkyl group having 1 to 20 carbon atoms), an alkyl group having 1 to 20 carbon atoms, either a monocyclic aromatic hydrocarbon group which may have a substituent or a ring-cumulated hydrocarbon group which may have a substituent and which is formed by linkage of a plurality of the aromatic hydrocarbon groups via a single bond or an unsaturated bond, a trialkylsilyl group, or a hydrogen atom,
      R₂ is a hydrogen atom or an alkyl group having 1 to 5 carbon atoms,
      R₃ is a hydrogen atom or an alkyl group having 1 to 20 carbon atoms, and
      n is an integer of 1 to m, provided that when Ar is a monocyclic aromatic hydrocarbon group, m is a number obtained by subtracting 1 from the number of carbon atoms that constitute the aromatic ring represented by Ar or, when Ar is a ring-cumulated hydrocarbon group, m is a number obtained by subtracting 1 from the number of carbon atoms that are not involved in a single bond or an unsaturated bond among the carbon atoms that constitute the aromatic ring,
      to react with
   (2) a vinyl compound represented by the following chemical formula 2: 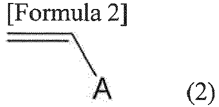
      wherein A is R₅ or a group represented by -COOR₅, where R₅ is an alkyl group having 1 to 20 carbon atoms or a halogenated alkyl group having 1 to 20 carbon atoms, in the presence of:
         (3-1) a metal complex represented by the following chemical formula 3 and (3-2) a compound in the class of percarboxylic acids or a hypervalent iodine compound, 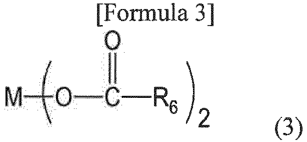
            wherein M is Pd, Pt, Au, Rh, or Ir and R₆ is an alkyl group having 1 to 5 carbon atoms, a phenyl group, or a phenyl group having, as a substituent, an alkyl group having 1 to 5 carbon atoms,
            to introduce a vinyl group derived from the vinyl compound onto a carbon atom which has no substituent and which is adjacent to the carbon atom to which the vinyl group of the vinyl aromatic compound is attached,
            the polyvalent vinyl aromatic compound being represented by the following chemical formula 4: 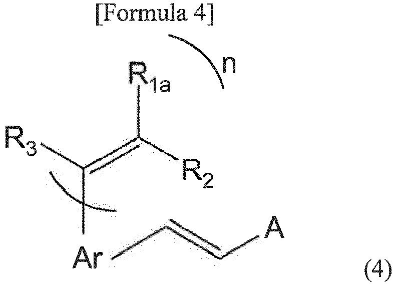
               wherein Ar is defined as stated above,
               R₁ₐ is R₁ or, when R₁ in the chemical formula 1 is a methyl group, R₁ₐ is -CH₂OC(=O)R₆, wherein R₁ and R₆ are defined as stated above, and
               R₂, R₃, A, and n are defined as stated above.
[2] The method according to [1], wherein M is Pd and R₆ is a methyl group or a phenyl group, in the metal complex of the chemical formula 3.
[3] The method according to [1] or [2], wherein the compound in the class of percarboxylic acids is a perbenzoate ester and the hypervalent iodine compound is diacyloxyiodobenzene.
[4] The method according to any one of [1] to [3], wherein, relative to 1 mol of the vinyl aromatic compound of the chemical formula 1, the vinyl compound of the chemical formula 2 is used in an amount of 2 to 5 mol, the metal complex of the chemical formula 3 is used in an amount of 0.1 to 0.5 mol, and the compound in the class of percarboxylic acids or the hypervalent iodine compound is used in an amount of 1 to 3 mol.
[5] The method according to any one of [1] to [4], wherein, in the vinyl aromatic compound of the chemical formula 1, Ar is a phenyl group, R₁ is a phenyl group, an alkyl group having 1 to 4 carbon atoms, a silyl group, or a group represented by -CH₂OC(=O)R₄ where R₄ is an alkyl group having 1 to 20 carbon atoms, and R₂ and R₃ are independently a hydrogen atom or a methyl group and wherein, in the vinyl compound of the chemical formula 2, A is a group represented by -COOR₅ where R₅ is an alkyl group having 1 to 20 carbon atoms.
[6] The method according to any one of [1] to [5], wherein the aforementioned reaction is performed in a carboxylic acid solvent.
[7] A method for producing a polyvalent vinyl aromatic compound comprising:
   a step of producing a polyvalent vinyl aromatic compound by allowing a vinyl aromatic compound represented by the following chemical formula 1a: 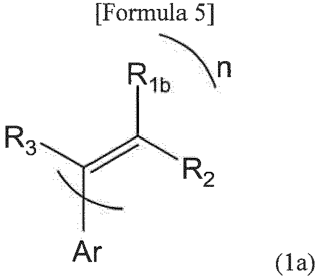
      wherein Ar and n are defined as stated above,
      R_{1b} is a methyl group or -CH₂OC(=O)R₄ wherein R₄ is an alkyl group having 1 to 20 carbon atoms,
      R₂ is a hydrogen atom or an alkyl group having 1 to 5 carbon atoms, and
      R₃ is a hydrogen atom or an alkyl group having 1 to 20 carbon atoms,
      to react with
   (2) a vinyl compound represented by the following chemical formula 2a: 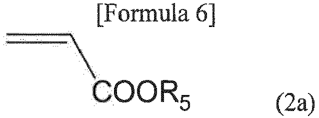
      wherein R₅ is an alkyl group having 1 to 20 carbon atoms or a halogenated alkyl group having 1 to 20 carbon atoms,
      in the presence of:
         (3-1) a metal complex represented by chemical formula 3 and (3-2) a percarboxylic acid or a hypervalent iodine compound,
            to introduce a vinyl group derived from the vinyl compound onto a carbon atom which has no substituent and which is adjacent to a carbon atom to which a vinyl group is attached in the vinyl aromatic compound,
            the second mentioned polyvalent vinyl aromatic compound being represented by the following chemical formula 4a: 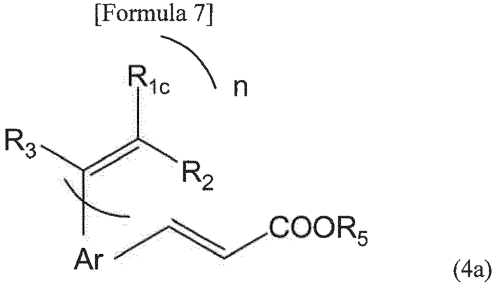
            wherein Ar and n are defined as stated above and R_{1c} is -CH₂OC(=O)R₄ or, when R_{1b} of the vinylbenzene compound of the chemical formula 1a is a methyl group, R_{1c} is -CH₂OC(=O)R₆;
            a step of reducing an ester group of the compound obtained by the introduction step to a hydroxy group;
            a step of oxidizing the hydroxy group of a compound obtained by the reduction step to an aldehyde group; and
            a step of converting the aldehyde group into a difluoromethyl group by reacting a compound obtained by the oxidation step and a compound represented by the following chemical formula 5: 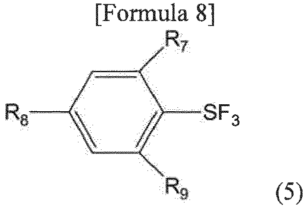
               wherein R₇ and R₉ are independently a hydrogen atom or an alkyl group having 1 to 8 carbon atoms and R₈ is a hydrogen atom, a halogen atom, or an alkyl group having 1 to 8 carbon atoms, and
               the first mentioned polyvalent vinyl aromatic compound being represented by the following chemical formula 6: 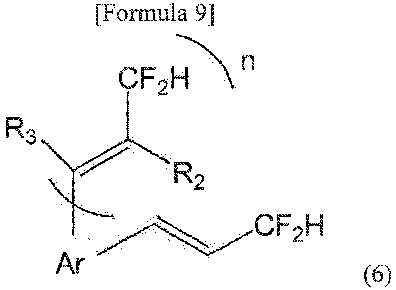 [0026] wherein Ar, R₂, R₃, and n are defined as stated above.
[8] A polyvalent vinyl aromatic compound represented by the following chemical formula 4: 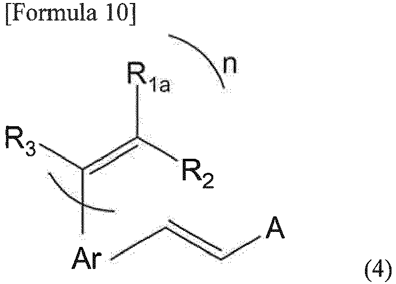 wherein Ar, R₁ₐ to R₃, A, and n are defined as stated above and the -CH₂=CH₂-A group is attached to a carbon atom that is adjacent to a carbon atom to which the -CHR₃=CR₁ₐR₂ group is attached.
[9] A polyvalent vinyl aromatic compound represented by the following chemical formula 4': 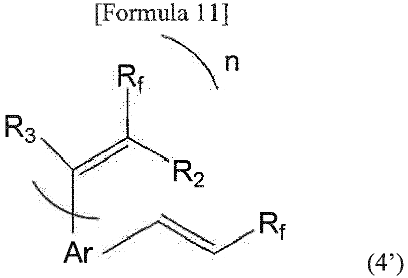 wherein Ar is defined as stated above, R_{f} are independently a difluoromethyl group or a perfluoromethyl group, R₂, R₃, and n are defined as stated above, and the -CH₂=CH₂-R_{f} group is attached to a carbon atom that is adjacent to a carbon atom to which the -CHR₃=CR_{f}R₂ group is attached.

### ADVANTAGEOUS EFFECTS OF INVENTION

The present invention provides a polyvalent vinyl aromatic compound having two or more vinyl groups on adjacent carbon atoms and a method for producing the compound.

### DESCRIPTION OF EMBODIMENTS

The present invention will be described in detail below. As used herein, "A to B" means values ranging from A to B (both ends inclusive).

### 1. Method for producing polyvalent vinyl aromatic compound

The production method of the present invention comprises the step of reacting (1) the vinyl aromatic compound represented by the chemical formula 1 and (2) the vinyl compound represented by the chemical formula 2 in the presence of (3-1) the metal complex and (3-2) the compound in the class of percarboxylic acids or the hypervalent iodine compound, as an oxidizing agent, to introduce the vinyl compound-derived vinyl group onto a carbon atom which has no substituent and which is adjacent to the carbon atom to which the vinyl group of the vinyl aromatic compound is attached.

In the vinyl aromatic compound, Ar is a monocyclic aromatic hydrocarbon group having a vinyl group on a carbon atom. Examples of the monocyclic aromatic hydrocarbon group include a phenyl group and a cyclopentadienyl group. In addition, examples of a heteroaromatic hydrocarbon group include a thiophenyl, thiopyranyl, pyrrolyl, pyrazolyl, imidazolyl, pyridyl, pyrazinyl, pyrimidinyl, furanyl, and pyranyl groups. Alternatively, Ar is a ring-cumulated hydrocarbon group formed by the linkage of a plurality of the aromatic hydrocarbon groups via a single bond or an unsaturated bond. In this case, the number of the linked monocycles is not limited, but is preferably 2 to 3, more preferably 2, in light of availability and the like. Examples of the ring-cumulated hydrocarbon group include a biphenylene, terphenylene, bithiophene, terthiophene, stilbenyl groups, and the like.

The vinyl aromatic compound represented by the chemical formula 1 includes vinyl group(s), the number of which is n. n is an integer of 1 to m. When Ar is a monocyclic aromatic hydrocarbon group, m is a number obtained by subtracting 1 from the number of the carbon atoms that constitute the aromatic ring represented by Ar and, when Ar is the ring-cumulated hydrocarbon group, m is a number obtained by subtracting 1 from the number of the carbon atoms that are not involved in a single bond or an unsaturated bond among the carbon atoms that constitute the aromatic ring. The carbon atoms that are not involved in a single bond or an unsaturated bond are carbon atoms that are not involved in a single bond or an unsaturated bond that links aromatic hydrocarbon groups, that is, carbon atoms that are not involved in the linkage of aromatic hydrocarbon groups. More specifically, when Ar is a phenyl group, n is 1 to 5; when Ar is a biphenylene group, n is 1 to 9; and when Ar is thiophene, n is 1 to 4. n is not limited, but is preferably 1 to 3, more preferably 1 to 2, in light of matters including the availability of the compound.

Those aromatic hydrocarbon groups may have a substituent on a carbon atom that is not adjacent to the carbon atom to which the vinyl group is attached. Examples of the substituent include an alkyl group having 1 to 20 carbon atoms, a group represented by -S-Ph (wherein Ph is a phenyl group or a halogenated phenyl group), a group represented by -C(=O)-Ph (wherein Ph is a phenyl group or a halogenated phenyl group), and a group represented by -SO₂-X (wherein X is an alkylamino group).

To simplify the description, the present invention will be described below with reference to an example in which Ar is a phenyl group and n is 1 in the vinyl aromatic compound represented by the chemical formula 1 (in this case, divinylbenzene can be produced as a polyvalent vinyl aromatic compound) and, as needed, a case where Ar is a heteroaromatic hydrocarbon group will be described. The reaction scheme is shown below. It is to be noted that the same applies to a case where Ar is a group other than a phenyl group.

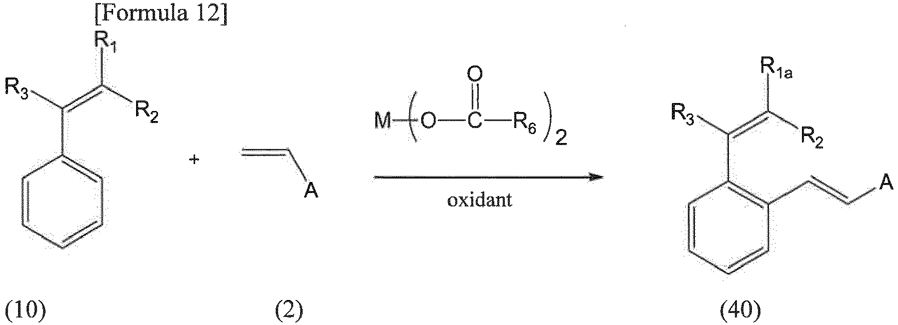

### 1-1. Components

### (1) Vinylbenzene compound

The vinylbenzene compound used as a starting material in the present invention is represented by chemical formula 10. Hereinafter, the vinylbenzene compound is also referred to as the "vinylbenzene compound 10".

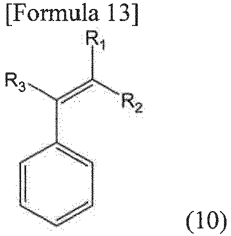

In the chemical formula 10, the phenyl group may have a substituent at a position other than the ortho position. The substituent may be selected arbitrarily as long as it does not interfere with the reactivity with the vinyl compound represented by the chemical formula 2 (hereinafter also referred to as the "vinyl compound 2"). As stated above, examples of the substituent include a group represented by -S-Ph (wherein Ph is a phenyl group or a halogenated phenyl group), a group represented by -C(=O)-Ph (wherein Ph is a phenyl group or a halogenated phenyl group), and a group represented by -SO₂-X (wherein X is an alkylamino group). Examples of the alkyl group having 1 to 20 carbon atoms include a methyl, ethyl, propyl, butyl, pentyl, hexyl, heptyl, octyl, nonenyl, decyl, undecyl, dodecyl, tridecyl, tetradecyl, pentadecyl, hexadecyl, heptadecyl, octadecyl, nonadecyl, and icosanyl groups. These groups include isomers and, for example, an isomer of a propyl group is a n-propyl group or an i-propyl group. If the substituent is too bulky, the reaction with the vinyl compound 2 can be less likely to proceed; hence, the substituent is preferably an alkyl group having 1 to 5 carbon atoms, such as a methyl, ethyl, propyl, butyl, or pentyl group.

R₁ is a group represented by -CH₂OC(=O)R₄ wherein R₄ is an alkyl group having 1 to 20 carbon atoms. Examples of the alkyl group having 1 to 20 carbon atoms are as stated above. If R₄ is too bulky, the reaction with the vinyl compound 2 can be less likely to proceed; hence, R₄ is preferably an alkyl group having 1 to 5 carbon atoms, such as a methyl, ethyl, propyl, butyl, or pentyl group.

R₁ may be the aforementioned alkyl group having 1 to 20 carbon atoms or a halogenated alkyl group having 1 to 20 carbon atoms.

R₁ may be an unsubstituted monocyclic aromatic hydrocarbon group or an unsubstituted ring-cumulated hydrocarbon group formed by the linkage of a plurality of the aromatic hydrocarbon groups via a single bond or an unsaturated bond. When R₁ is an unsubstituted monocyclic aromatic hydrocarbon group or an unsubstituted ring-cumulated hydrocarbon group, the reaction is likely to proceed. In this case, R₁ is particularly preferably an unsubstituted phenyl group. When R₁ is an aromatic hydrocarbon group having a substituent or a ring-cumulated hydrocarbon group having a substituent, the substituent is preferably a group that functions as a donor. When R₁ is such a group, a compound that is useful as a luminescent material can be produced. In this case, examples of the donor substituent include a diphenylamino, dialkylamino, carbazoyl, oxadiazole, pyrazolyl, nitro groups, and the like, but are not limited thereto. Meanwhile, when the substituent present on the aromatic hydrocarbon group or the ring-cumulated hydrocarbon group is too bulky, the reaction with the vinyl compound 2 can be less likely to proceed. From this viewpoint, the substituent is preferably an alkyl group having 1 to 5 carbon atoms. A plurality of the substituents may be present on the aromatic hydrocarbon group or the ring-cumulated hydrocarbon group.

R₁ may be a trialkylsilyl group. A trialkylsilyl group is a group formed by the linkage of three alkyl groups to a silicon atom. The alkyl group is preferably as stated for R₄. Examples of the trialkylsilyl group include a trimethylsilyl (TMS) group, a triethylsilyl (TES) group, a tert-butyldimethylsilyl (TBS) group, and a triisopropylsilyl (TIPS) group.

R₁ may be a hydrogen atom, but in this case, vinyl group polymerization is potentially likely to occur; hence, R₁ is preferably any one of the substituents described above.

When R₁ is a group represented by -CH₂OC(=O)R₄ among the aforementioned groups, there is the advantage that it can be easily converted into a fluoromethyl group, as mentioned later. A fluoromethyl group functions as a donor or an acceptor in a luminescent compound.

When R₁ is a methyl group, it is oxidized upon the reaction with the vinyl compound 2 and a group represented by -CH₂OC(=O)R₆ is formed. More specifically, a hydrogen of the methyl group is replaced by the metal complex-derived OC(=O)R₆ group. Hence, when R₁ is a methyl group, there is the advantage that it can be easily converted into a group represented by -CH₂OC(=O)R₆.

Aside from the aforementioned groups, R₁ may be a nitrile group or an alkylate group having 1 to 20 carbon atoms. The alkyl group having 1 to 20 carbon atoms is as described above.

R₂ is a hydrogen atom or an alkyl group having 1 to 5 carbon atoms. Specific examples of the alkyl group having 1 to 5 carbon atoms are as stated above, and the alkyl group may be a halogenated alkyl group. If R₂ is bulky, steric hindrance is high and the reaction with the vinyl compound 2 can be less likely to proceed; hence, R₂ is preferably a methyl group or an ethyl group, more preferably a hydrogen atom.

Aside from the aforementioned groups, R₂ may be a nitrile group or an alkylate group having 1 to 20 carbon atoms. The alkyl group having 1 to 20 carbon atoms is as described above.

R₃ is a hydrogen atom or an alkyl group having 1 to 20 carbon atoms. Specific examples of the alkyl group having 1 to 20 carbon atoms are as stated above. Since the distance of R₃ to the ortho position is longer than that of R₂, R₃ is less likely to inhibit the reaction with the vinyl compound 2. Hence, R₃ may be an alkyl group having 1 to 20 carbon atoms or a halogenated alkyl group having 1 to 20 carbon atoms. However, when R₃ is too bulky, it is likely to inhibit the aforementioned reaction and hence, R₃ is preferably an alkyl group having 1 to 5 carbon atoms or a halogenated alkyl group having 1 to 5 carbon atoms, more preferably a methyl group or an ethyl group, still more preferably a hydrogen atom. Aside from the foregoing groups, R₃ may be a nitrile group.

Cases where Ar is a phenyl group are described above and, meanwhile, when Ar is a heteroaromatic hydrocarbon group, for example, a vinyl thiophene compound shown below may be used as a compound represented by the chemical formula 1.

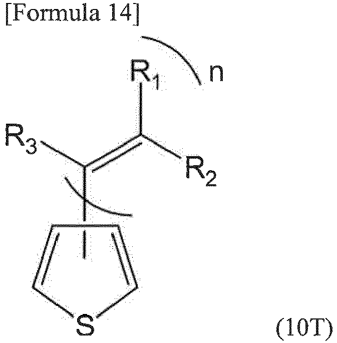

In this formula, a vinyl group is attached to a carbon atom of the thiophenyl group and R₁ to R₃ in the vinyl group are defined as stated above. The thiophenyl group may have a substituent as described above on a carbon atom that is not adjacent to the carbon atom having the vinyl group. n is defined as stated above.

Specific examples of such a vinyl thiophene compound include the compounds shown below.

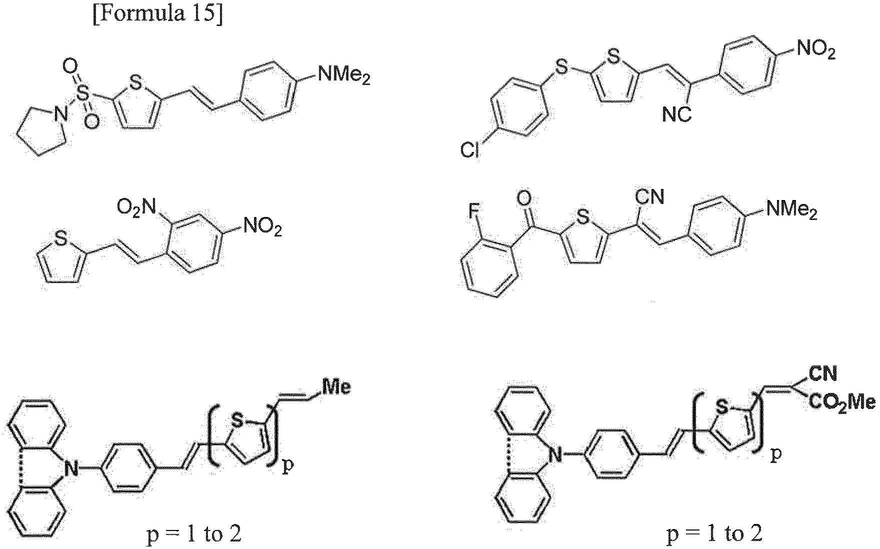

### (2) Vinyl compound of chemical formula 2

The vinyl compound used as a starting material in the present invention is represented by the chemical formula 2.

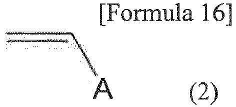

In the chemical formula 2, A is R₅, which is an alkyl group having 1 to 20 carbon atoms or a halogenated alkyl group having 1 to 20 carbon atoms. Specific examples of the alkyl group are as described above for the vinylbenzene compound 10, and if R₅ is too bulky, the reaction with the vinylbenzene compound 10 can be less likely to proceed. Hence, R₅ is preferably an alkyl group having 1 to 5 carbon atoms or a halogenated alkyl group having 1 to 5 carbon atoms. When R₅ is a halogenated alkyl group such as a perfluoromethyl group or a difluoromethyl group (that is, A is such a halogenated alkyl group), a compound having a fluoroalkylated vinyl group can be obtained, and the compound is useful as a luminescent material and can also be used as an anchor to the titanium oxide in materials for dye-sensitized solar cells.

Alternatively, A is an ester group represented by -COOR₅. If R₅ is too bulky, the reaction with the vinylbenzene compound can be less likely to proceed, nor can vinyl compounds having the group be easily obtained. Hence, when A is -COOR₅, R₅ is preferably an alkyl group having 1 to 5 carbon atoms, more preferably a methyl group or an ethyl group. As mentioned above, when A is a halogenated alkyl group, a compound useful as a luminescent material or the like can be obtained, and also, when A is an ester group, there is the advantage that since it can be easily converted into a fluoromethyl group, as mentioned later, a compound having a fluoroalkylated vinyl group is likely to be obtained.

The vinyl compound 2 is preferably used in an amount of 2 to 5 mol, more preferably 2.5 to 3.5 mol, relative to 1 mol of the aforementioned benzene compound. When the molar number of the vinyl compound falls within the range, reaction proceeds efficiently.

### (3) Metal complex of chemical formula 3

The metal complex used as a catalyst in the present invention is represented by the chemical formula 3.

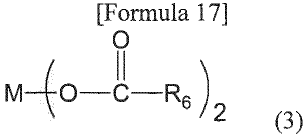

M is a divalent metal atom, which is Pd (palladium), Pt (platinum), Au (gold), Rh (rhodium), or Ir (iridium). M is particularly preferably Pd in terms of availability and the like.

R₆ is an alkyl group having 1 to 5 carbon atoms, a phenyl group, or a phenyl group having, as a substituent, an alkyl group having 1 to 5 carbon atoms. The alkyl group having 1 to 5 carbon atoms is as described above. Specific examples of such a metal complex (for example, in the case where M is Pd) include palladium acetate (R₆ is a methyl group), palladium propionate (R₆ is an ethyl group), palladium butyrate (R₆ is a butyl group), palladium valerate (R₆ is a pentyl group), and palladium benzoate (R₆ is a phenyl group). Among these groups, R₆ is preferably a methyl group or a phenyl group in terms of availability.

In light of reaction efficiency and cost, the metal complex is preferably used in an amount of 0.1 to 0.5 mol, more preferably 0.15 to 0.25 mol, relative to 1 mol of the vinylbenzene compound 10.

### (4) Oxidizing agent

In the present invention, a compound in the class of percarboxylic acids or a hypervalent iodine compound is used as an oxidizing agent. Since reaction mechanisms differ depending on the oxidizing agents used, it is preferable not to use both oxidizing agents together. The oxidizing agents will be described below.

### 1) Compound in the class of percarboxylic acids

In the present invention, a compound in the class of percarboxylic acids is used as an oxidizing agent. The term "compound in the class of percarboxylic acids" refers to a percarboxylic acid or a percarboxylic acid ester. A known compound in the class of percarboxylic acids may be used and examples include perbenzoate esters such as butyl perbenzoate and benzoyl peroxide.

With regard to the reaction mechanism, which will be described later, the aforementioned metal complex used as a catalyst is reduced along with the reaction between the vinylbenzene compound 10 and the vinyl compound 2. Hence, the reduced metal complex is oxidized with a compound in the class of percarboxylic acids and regenerated. The amount of a compound in the class of percarboxylic acids that is used is preferably 1 to 3 mol relative to 1 mol of the vinylbenzene compound 10. When the used amount is below the lower limit, the aforementioned oxidation reaction can possibly not be performed sufficiently and, in contrast, when the used amount is above the upper limit, a side reaction such as polymerization of the vinyl compound 2 is likely to occur.

### 2) Hypervalent iodine compound

In the present invention, a hypervalent iodine compound is used as an oxidizing agent. The term "hypervalent iodine compound" is a general term for three-, five-, seven-, or eight-coordinate iodine compounds. Specific examples of the hypervalent iodine compound include diacyloxyiodobenzene (a three-coordinate iodine compound) such as iodophenyl diacetate and DMP (Dess-Martin Periodinane) (a five-coordinate iodine compound).

With regard to the reaction mechanism, which will be described later, a hypervalent iodine compound oxidizes the central metal of the metal complex from the +2 valence state to the +4 valence state and the quadrivalent metal complex is assumed to accelerate the reaction between the vinylbenzene compound 10 and the vinyl compound 2. The amount of a hypervalent iodine compound is preferably 1 to 3 mol relative to 1 mol of the vinylbenzene compound 10. When the amount of a hypervalent iodine compound used is below the lower limit, the aforementioned oxidation reaction can possibly not be performed sufficiently and, in contrast, when the amount is above the upper limit, a side reaction such as polymerization of the vinyl compound 2 is likely to occur.

### 1-2. Reaction conditions

### (1) Solvent

A solvent is selected as appropriate so that the starting compounds and the catalyst can be dissolved in the solvent. In the present invention, a solvent having a carboxyl group such as acetic acid or propionic acid, that is, a carboxylic acid solvent, is preferred since it is superior in affinity with a metal complex.

### (2) Temperature and time

The reaction temperature is preferably 50 to 150°C. When the temperature is below the lower limit, the reaction can possibly not proceed sufficiently and, in contrast, when the temperature is above the upper limit, a side reaction such as polymerization of the vinyl group of the vinylbenzene compound 10 or the vinyl compound 2 can possibly occur.

The reaction time may be adjusted as appropriate to enable the reaction to be completed. For example, it is preferable to adjust the reaction temperature to 80 to 120°C and the reaction time to about 5 to 24 hours.

To prevent the deterioration of the starting materials, the reaction is preferably performed under an atmosphere of inert gas such as argon or nitrogen.

### 1-3. Product

The divinylbenzene compound obtained by the aforementioned method is represented by chemical formula 40. The reaction mechanism will be described in the next section.

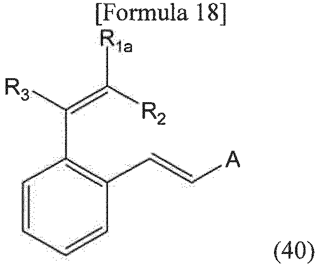

R₁ₐ is R₁ or -CH₂OC(=O)R₆, which is defined as stated above. As mentioned above, when R₁ is a methyl group in the vinylbenzene compound 10, which is a starting material, the methyl group is oxidized to -CH₂OC(=O)R₆. R₆ is derived from the -OC(=O)R₆ group in the metal complex. R₂, R₃, and A are defined as stated above.

In the compound 40, the -CH₂=CH₂-A group is attached to a carbon atom that is adjacent to the carbon atom to which the -CHR₃=CR₁ₐR₂ group is attached. When a benzene compound having a plurality of vinyl groups (-CHR₃=CR₁R₂ groups) is used as the starting compound 10, the -CH₂=CH₂-A group is introduced onto a carbon atom that is adjacent to carbon atoms to which the -CHR₃=CR₁ₐR₂ groups are attached. For example, when a benzene compound having two vinyl groups (-CHR₃=CR₁R₂ groups) on carbon atoms at the 1- and 4-positions, respectively, is used as the starting compound 10, the -CH₂=CH₂-A group is introduced onto one or more carbon atoms at the position(s) among the 2-, 3-, 5-, and 6-positions of the benzene ring. When a thiophene compound having two vinyl groups (-CHR₃=CR₁R₂ groups) on the carbon atoms at the 2- and 5-positions is used as the starting compound 10, the -CH₂=CH₂-A group is introduced onto one or more carbon atoms at the position(s) among the 3- and 4-positions of the thiophene ring.

### 1-4. Reaction mechanism

### (1) Case using compound in the class of percarboxylic acids

The mechanism of this reaction is illustrated by the scheme provided below. To simplify the description, the scheme shows an example in which R₂ and R₃ in the vinylbenzene compound 10 are each a hydrogen atom, A in the vinyl compound 2 is -COOR₅, M in the metal complex is Pd, and the compound in the class of percarboxylic acids is t-butyl perbenzoate.

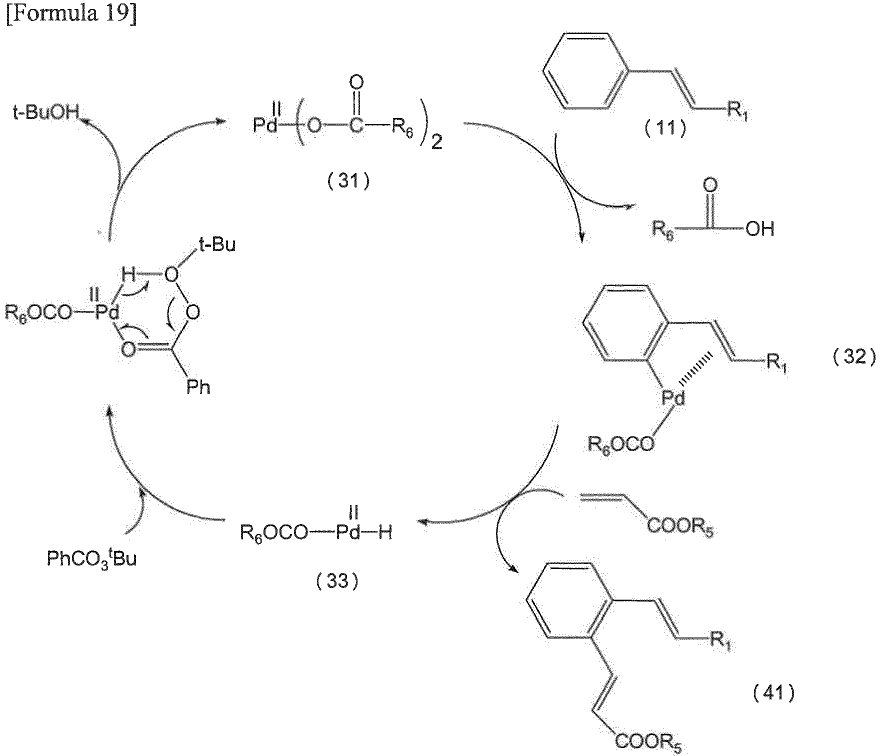

First, a vinylbenzene compound (11) and a metal complex (31) are reacted to generate an intermediate (32). In this reaction, Pd is coordinated to the vinyl group of the vinylbenzene compound (11) and attached to the ortho position of the vinylbenzene compound (11). Subsequently, the intermediate (32) and a vinyl compound are reacted and a compound (33) is eliminated to generate a divinylbenzene compound (41), which is an intended compound. The compound (33) is oxidized with the percarboxylic acid to regenerate the original-state metal complex (31). In this cycle, Pd remains divalent.

### (2) Case using hypervalent iodine compound

The mechanism of this reaction is illustrated by the scheme provided below. To simplify the description, the scheme shows an example in which R₂ and R₃ in the vinylbenzene compound 10 are each a hydrogen atom, A in the vinyl compound 2 is -COOR₅, M in the metal complex is Pd, and the hypervalent iodine compound is diacetoxyiodobenzene.

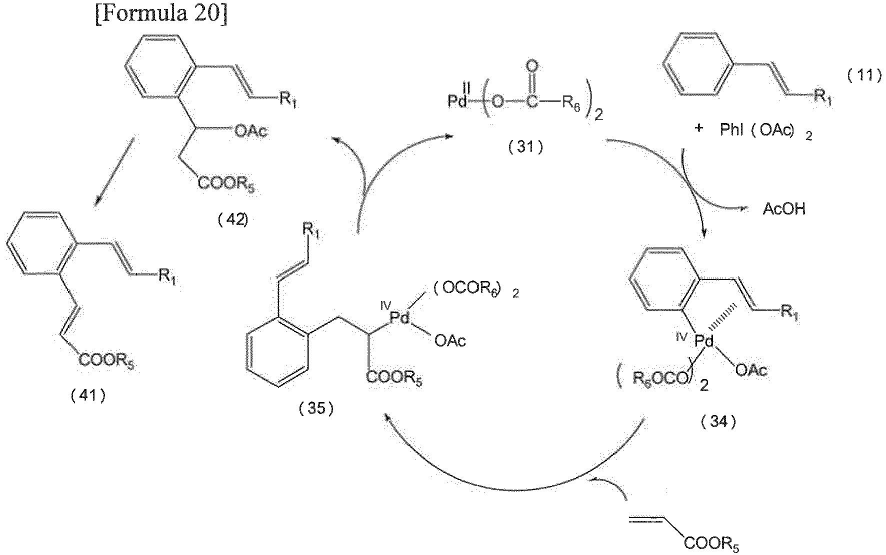

First, a vinylbenzene compound (11), a metal complex (31), and diacetoxyiodobenzene are reacted to generate an intermediate (34). In this reaction, Pd is coordinated to the vinyl group of the vinylbenzene compound (11) and attached to the ortho position of the vinylbenzene compound (11). Subsequently, the intermediate (34) and a vinyl compound are reacted to generate an intermediate (35). From this intermediate, an intermediate (42) is eliminated to regenerate the original-state metal complex (31). From the intermediate (42), acetic acid is eliminated to generate a divinylbenzene compound (41), which is an intended compound. In this cycle, Pd changes as follows: divalent Pd → tetravalent Pd → divalent Pd.

As mentioned above, through either reaction mechanism, Pd is coordinated to the vinyl group of the vinylbenzene compound 10, and an intermediate in which Pd is attached to the ortho position of the vinylbenzene compound 10 is formed. Hence, in the present invention, the vinyl group derived from the vinyl compound 2 can be introduced at the ortho position efficiently. As a result, in the aforementioned schemes, a divinylbenzene compound 4 represented by the chemical formula 4 can be produced.

### 2. Method for producing benzene compound having fluoromethylated vinyl groups

In the present invention, a benzene compound having fluoromethylated vinyl groups that is useful as a luminescent material or the like can be produced by fluoromethylation of a specific divinylbenzene compound having vinyl groups that is obtained by the aforementioned production methods. Specifically, the method for producing the benzene compound having fluoromethylated vinyl groups comprises:
1) a step of synthesizing a divinylbenzene 40a by reacting a vinylbenzene compound 10a and an alkyl acrylate 2a as a vinyl compound in the presence of the aforementioned metal complex and oxidizing agent to introduce a group derived from the alkyl acrylate 2a to the vinylbenzene compound 10a at the ortho position,
2) a reduction step to give a compound 40b by reducing the ester group of the compound 40a, which is obtained by the synthesis step, to a hydroxy group,
3) an oxidation step to give a compound 40c by oxidizing the hydroxy group of the compound 40b, which is obtained by the reduction step, to an aldehyde group, and
4) a fluorination step to give a compound 60 by reacting the compound 40c, which is obtained by the oxidation step, and a fluorinating agent to convert the aldehyde group into a difluoromethyl group.

The reaction scheme of this production method is shown below. In the scheme, R_{1b} and R₂ to R₆ are as described above.

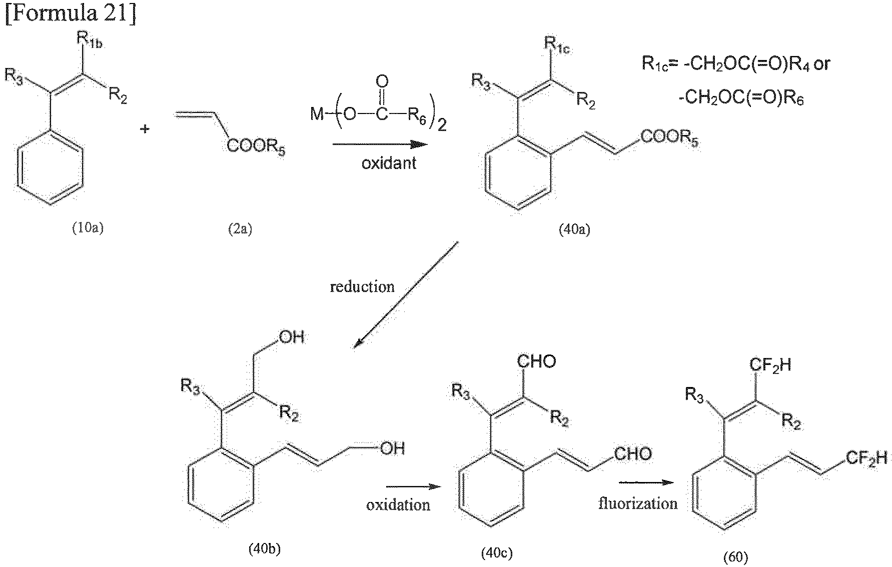

### 2-1. Step of synthesizing specific divinylbenzene compound

In this step, a divinylbenzene (the chemical formula 40a) is synthesized. The divinylbenzene 40a can be synthesized by use of the vinylbenzene compound 10a and the alkyl acrylate 2a as starting materials in the production method mentioned above.

In the scheme mentioned above, R_{1b} is a methyl group or -CH₂OC(=O)R₄ wherein R₄ is an alkyl group having 1 to 20 carbon atoms. R_{1c} is a group represented by -CH₂OC(=O)R₄ or -CH₂OC(=O)R₆. R₄ is an alkyl group having 1 to 20 carbon atoms, R₆ is an alkyl group having 1 to 5 carbon atoms, a phenyl group, or a phenyl group having, as a substituent, an alkyl group having 1 to 5 carbon atoms. R₂, R₃, and phenyl group are as described above.

### 2-2. Reduction step

In this step, the ester group of the divinylbenzene compound 40a is reduced to a hydroxy group to give the compound 40b.

### (1) Reducing agent

A known reducing agent may be used, but an aluminum-based reducing agent such as diisobutyl aluminum hydride is preferred in terms of availability and activity. The amount of a reducing agent used may be a known amount, but is preferably 3 to 10 mol relative to 1 mol of the compound 40a.

### (2) Conditions

The reaction temperature may be set as is known but, to suppress any side reactions, the temperature is generally a low temperature of -100 to -30°C, preferably -90 to -70°C. The reaction time may be adjusted as appropriate depending on the progress of the reaction, but is generally 1 to 10 hours.

### 2-3. Oxidation step

The hydroxy group of the compound 40b, which is obtained by the reduction step, is oxidized with an oxidizing agent to an aldehyde group to give the compound 40c.

### (1) Oxidizing agent

A known oxidizing agent may be used, but a metal oxide-based oxidizing agent such as manganese dioxide is preferred in terms of availability and activity. The amount of an oxidizing agent used may be a known amount, but is preferably 10 to 30 mol relative to 1 mol of the compound 40b.

### (2) Conditions

The reaction temperature may be set as is known but, to suppress any side reactions, the temperature is preferably 10 to 80°C, more preferably about room temperature (20 to 30°C). The reaction time may be adjusted as appropriate depending on the progress of the reaction, but is generally 10 to 30 hours. The oxidation step may be performed without interruption after the reduction step.

### 2-4. Fluorination step

In this step, the aldehyde group of the compound 40c, which is obtained by the oxidation step, is fluorinated with a fluorinating agent to give the compound 60.

### (1) Fluorinating agent

As the fluorinating agent, a compound represented by the chemical formula 5 may be used:

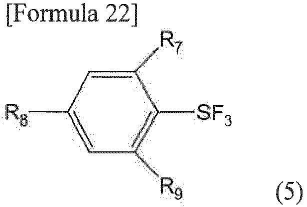

R₇ and R₉ are independently a hydrogen atom or an alkyl group having 1 to 8 carbon atoms, and R₈ is a hydrogen atom, a halogen atom, or an alkyl group having 1 to 8 carbon atoms. Specific examples of the alkyl group having 1 to 8 carbon atoms are as stated above. In the present invention, R₇ and R₉ are each preferably a methyl group in terms of availability and reaction activity. R₈ is preferably a tertiary alkyl group having 1 to 8 carbon atoms, more preferably a t-butyl group, in terms of reaction activity. Examples of such a compound (R₇ and R₉ are each a methyl group and R₈ is a t-butyl group) include FLUOLEAD (registered trade name) from Ube Industries, Ltd. The amount of a fluorinating agent used may be a known amount, but is preferably 2 to 10 mol relative to 1 mol of the compound 40c.

### (2) Conditions

To suppress any side reactions, the reaction temperature is preferably 10 to 80°C, more preferably about room temperature (20 to 30°C). The reaction time may be adjusted as appropriate depending on the progress of the reaction, but is generally 10 to 30 hours.

To accelerate the fluorination reaction, an alcohol is preferably used as a reaction accelerator in this step. The alcohol is preferably an alkyl alcohol having 1 to 5 carbon atoms and especially, the alcohol is more preferably methanol or ethanol in terms of availability. Alcohol reacts with a fluorinating agent used in the present invention to generate hydrogen fluoride (HF) and the HF accelerates the fluorination reaction of the compound 40c in the step. This mechanism is not limited, but it is speculated that since a fluorine atom of a fluorinating agent used in the present invention and the HF are hydrogen bonded to increase the cationicity of the sulfur atom, the tendency for an oxygen atom of the compound 40c to attack the sulfur atom is increased (see the scheme shown below). It is conjectured that after oxygen atoms attack the sulfur atom, the oxygen atoms and carbon atoms attached thereto are split and also two atomic fluorine anions are released from the fluorinating agent and attached to the carbon atoms to form difluoromethyl groups (see J. Am. Chem. Soc., 132, p. 18199 (2010)).

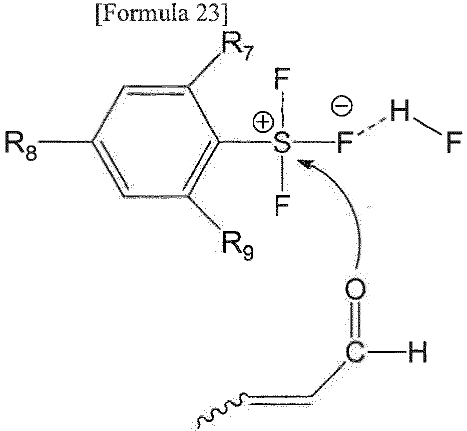

To obtain the reaction-accelerating effect, the amount of an alcohol used is preferably 0.01 to 0.05 mol relative to 1 mol of a fluorinating agent.

In accordance with the foregoing steps, the compound 60, which has fluoromethylated vinyl groups, can be produced.

A step of fluorinating the formylvinyl groups of the compound 40c is described above. Alternatively, for example, the hydroxymethyl vinyl groups of the compound 40b may be fluorinated to produce a compound having perfluoromethylated vinyl groups.

### 3. Polyvinylbenzene compound

### (1) Structure

As stated above, when Ar is a phenyl group, a polyvinylbenzene compound occurs as a polyvalent aromatic compound according to the present invention. The polyvinylbenzene compound is represented by, for example, the following chemical formula 40:

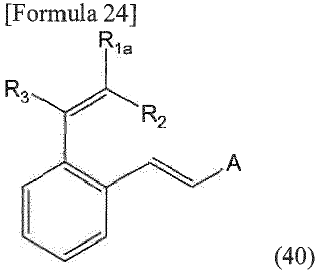

R₁ₐ to R₃, A, and phenyl group are as stated above.

The polyvinylbenzene compound having fluoromethylated vinyl groups according to the present invention is represented by the following chemical formula 40':

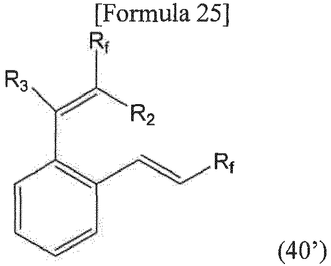

R_{f} are independently a difluoromethyl group or a perfluoromethyl group. When the compound is used as a luminescent material, the difluoromethyl group can function as an acceptor or a donor, and the perfluoromethyl group can function as an acceptor. In the present invention, both of R_{f} are preferably difluoromethyl groups.

### (2) Use

As mentioned above, the polyvalent vinyl aromatic compound of the present invention is useful as a luminescent material or a liquid crystal material in the field of displays or as a material for dye-sensitized solar cells.

### EXAMPLES

The present invention will be described in more detail below with reference to Examples and Comparative Examples.

### [Example 1]

Through the reaction shown below, a divinylbenzene compound was produced.

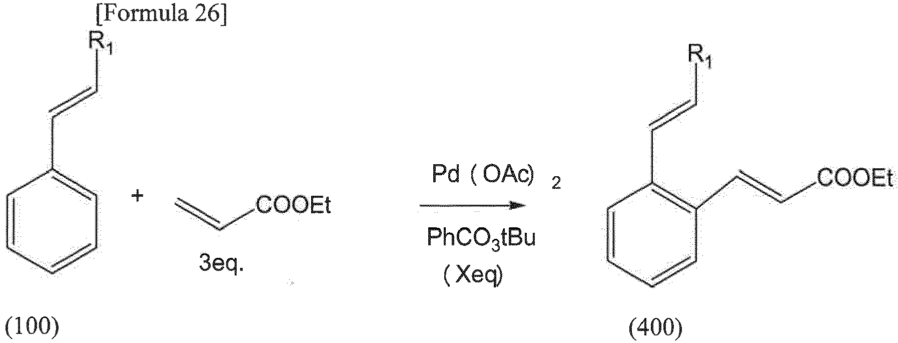

The details are as stated below.

### [Example 1-1]

In acetic acid (Kanto Chemical Co., Inc.), 4.5 mg of Pd(OAc)₂ (a product of Sigma-Aldrich) and 38 µL (2.0 mmol) of t-butyl perbenzoate (a product of Sigma-Aldrich) were dissolved to prepare an acetic acid solution.

In a flask, 1.0 mmol of a vinylbenzene compound represented by chemical formula 100 wherein R₁ is a methyl group and 33 µL (3.0 mmol) of ethyl acrylate were charged. Subsequently, the acetic acid solution was charged in the flask at room temperature under an argon atmosphere. This step was performed such that the amount of Pd(OAc)₂ was 20 mol % relative to the vinylbenzene compound. The flask was heated to adjust the internal temperature to 100°C and the mixture was stirred for 12 hours to perform a reaction. After that, the reaction mixture was cooled and a saturated aqueous solution of NaCO₃ was charged in the flask to stop the reaction. An organic phase was separated from the mixture in the flask. The remaining aqueous phase was extracted with ether three times to collect an organic phase. The previously separated organic phase and the organic phase obtained by the extraction with ether were combined, and the combined organic phase was washed with a saturated aqueous solution of NaCl and then dried over MgSO₄. Further, the organic phase was placed under reduced pressure to remove the organic solvent. The resulting crude product was purified by silica gel chromatography. In this way, (E)-ethyl 3-(2-((E)-3-acetoxyprop-1-en-1-yl)phenyl)acrylate, which is a divinylbenzene compound represented by chemical formula 401, was produced. The ¹H-NMR spectrum of the compound obtained (which was measured with AV300M from Bruker at 300 MHz using CDCl₃) was as shown below. The NMR result revealed a yield of 8%. 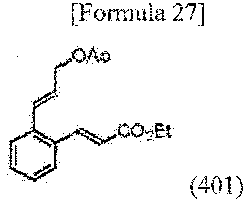 ¹H NMR (300MHz, CDCl₃) δ 1.34 (t, J = 7.14 Hz, 3 H), 2.10 (s, 3H), 4.27 (q, J = 7.11 Hz, 2 H), 4.77 (d, J J = 6.24 Hz, 2H), 6,17 (dt, J = 15.9, 6.30 Hz, 1H), 6.34 (d, J = 15.9 Hz, 1H), 6.99 (d, J = 15.8 Hz, 1H), 7.28 7.54 (m, 4 H), 8.00 (d, J = 15.9 Hz, 1H).

### [Example 1-2]

A divinylbenzene compound 402 was obtained in the same manner as in Example 1 - 1 except that a compound wherein R₁ is a phenyl group was used as the vinylbenzene compound 100. The result is shown in Table 1. The ¹H-NMR spectrum of the compound obtained (which was measured with AV300M from Bruker at 300 MHz using CDCl₃) was as shown below. 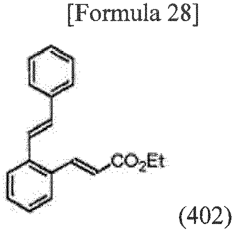 ¹H NMR (300MHz, CDCl₃) δ1.84 (t, J = 7.14 Hz, 3H), 4.27 (q, J = 7.11 Hz, 2 H), 6.99 (d, J = 16.2 Hz, 1H), 7.13 -7.58 (m, 9H), 8.13 (d, J = 1.65 Hz, 1H).

### [Example 1-3]

A divinylbenzene compound 403 was obtained in the same manner as in Example 1-1 except that a compound wherein R₁ is a t-butyl group was used as the vinylbenzene compound 100. The result is shown in Table 1. The ¹H-NMR spectrum of the compound obtained (which was measured with AV300M from Bruker at 300 MHz using CDCl₃ and TMS as an internal standard) was as shown below. 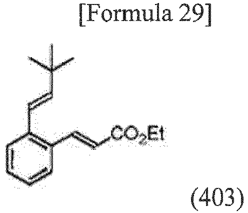 ¹H NMR (800MHz, CDCl₃) δ 1.15 (s, 9H), 1.34 (t, J = 7.11, 3H), 4.27 (q, J= 7.14 Hz, 2 H), 6.07 (d, J = 15.9 Hz, 1 H), 6.34 (d, J = 15.9 Hz, 1 H), 6.59 (d, J = 15.9 Hz, 1H), 7.20-7.54 (m, 4 H), 8.03 (d, J = 15.9 Hz, 1H).

### [Example 1-4]

The divinylbenzene compound 401 was obtained in the same manner as in Example 1-1 except that a compound wherein R₁ is an acetoxymethyl group was used as the vinylbenzene compound 100. The ¹H-NMR spectrum of the compound obtained was the same as that of the compound obtained in Example 1-1. The result is shown in Table 1.

### [Example 1-5]

A reaction was performed in the same manner as in Example 1-1 except that the amount of t-butyl perbenzoate was 5.0 mmol. As a result, the divinylbenzene compounds 401 were obtained, but a polymer formed by polymerization of the vinyl groups contained in some of the compounds was produced as a by-product. The yield was not calculated.

### [Examples 1-6 to 1-9]

Reactions were performed in the same manner as in Example 1-1 except that the solvents shown in Table 1 were substituted for acetic acid. As a result, divinylbenzene compounds were obtained in minute yields. In these examples, the yields were not calculated.

**[Table 1]**

| | | R₁ | X (equivalent) | Solvent | Yield (%) |
|---|---|---|---|---|---|
| Example 1 | 1 | methyl group | 2 | acetic acid | 8 |
| | 2 | phenyl group | 2 | acetic acid | 16 |
| | 3 | t-butyl group | 2 | acetic acid | 12 |
| | 4 | acetoxymethyl group | 2 | acetic acid | 10 |
| | 5 | methyl group | 5 | acetic acid | Not calculated |
| | 6 | methyl group | 2 | DMF | Not calculated |
| | 7 | methyl group | 2 | 1,4-dioxane | Not calculated |
| | 8 | methyl group | 2 | acetonitrile | Not calculated |
| | 9 | methyl group | 2 | DMSO | Not calculated |

### [Comparative Example 1-1]

A reaction was performed in the same manner as in Example 1-1 except that a compound represented by the following chemical formula that has an ethylenyl group was substituted for the vinylbenzene compound 100. However, any reaction of substitution with the ethyl acrylate-derived group at the ortho position did not occur.

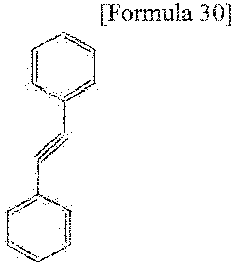

### [Comparative Example 1-2]

A reaction was performed in the same manner as in Example 1-1 except that a compound represented by the following chemical formula that has a nitrile group was substituted for the vinylbenzene compound 100. However, any reaction of substitution with the ethyl acrylate-derived group at the ortho position did not occur.

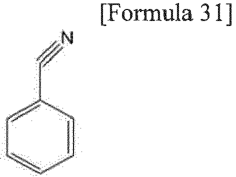

### [Example 2]

Through the reaction shown below, a divinylbenzene compound was produced.

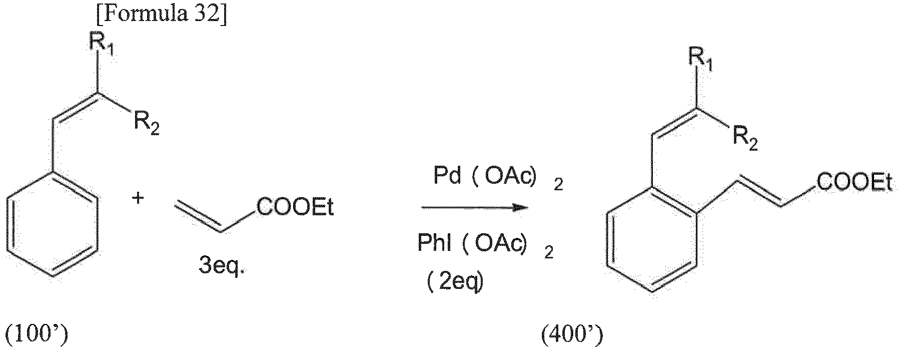

The details are as stated below.

### [Example 2-1]

In acetic acid (Kanto Chemical Co., Inc.), 4.5 mg of Pd(OAc)₂ (a product of Sigma-Aldrich) and 64 mg (2.0 mmol) of iodophenyl diacetate (a product of Sigma-Aldrich) were dissolved to prepare an acetic acid solution.

In a flask, 1.0 mmol of a vinylbenzene compound represented by chemical formula 100' wherein R₁ is a methyl group and R₂ is a hydrogen atom and 33 µL (3.0 mmol) of ethyl acrylate were charged. Subsequently, the acetic acid solution was charged in the flask at room temperature under an argon atmosphere. This step was performed such that the amount of Pd(OAc)₂ was 10 mol % relative to the vinylbenzene compound. The flask was heated to adjust the internal temperature to 100°C and the mixture was stirred for 12 hours to perform a reaction. After that, the reaction mixture was cooled and a saturated aqueous solution of NaCO₃ was charged in the flask to stop the reaction. An organic phase was separated from the mixture in the flask. The remaining aqueous phase was extracted with ether three times to collect an organic phase. The previously separated organic phase and the organic phase obtained by the extraction with ether were combined, and the combined organic phase was washed with a saturated aqueous solution of NaCl and then dried over MgSO₄. Further, the organic phase was placed under reduced pressure to remove the organic solvent. The resulting crude product was purified by silica gel chromatography. In this way, (E)-ethyl 3-(2-((E)-3-acetoxyprop-1-en-1-yl)phenyl)acrylate, which is a divinylbenzene compound represented by the chemical formula 401, was produced. The yield was 17%. The ¹H-NMR spectrum of the compound obtained was the same as that of the compound obtained in Example 1-1.

### [Examples 2-2 and 2-3]

The divinylbenzene compounds 401 were produced in the same manner as in Example 2-1 except that the amounts of Pd(OAc)₂ were 20 mol % and 30 mol %, respectively. The ¹H-NMR spectra of the compounds obtained were the same as the spectrum of the compound obtained in Example 1-1. The results are shown in Table 2.

### [Examples 2-4 to 2-6]

Divinylbenzene compounds were produced in the same manner as in Example 2-1 except that the vinylbenzene compounds shown in Table 2 were used and that the amounts of Pd(OAc)₂ were 20 mol %. The obtained compounds exhibited the same ¹H-NMR spectra as those of the compounds obtained in Examples 1-2, 1-3, and 1-1, respectively, and were confirmed to be identical to the compounds 402, 403, and 401, respectively.

### [Example 2-7]

A divinylbenzene compound was produced in the same manner as in Example 2-1 except that the vinylbenzene compound shown in Table 2 was used and that the amount of Pd(OAc)₂ was 20 mol %. As a result, a divinylbenzene compound 404 was obtained. The ¹H-NMR spectrum of the obtained compound was as shown below. 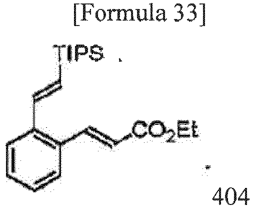 ¹H NMR (300MHz, CDCl₃) δ 1.11-1.1.23 (m, 18 H), 1.34 (t, J = 7.14 Hz, 3 H), 4.27 (q, J = 7.11 Hz, 2 H), 6.26 (d, J = 19.2, 1H), 6.36 (d, J = 15.9 Hz, 1 H), 6.96 (d, J = 18.0 Hz, 1 H), 7.31-7.55 (m, 4 H), 8.06 (d, J = 15.9 Hz, 1H).

### [Example 2-8]

A divinylbenzene compound was produced in the same manner as in Example 2-1 except that the vinylbenzene compound shown in Table 2 was used and that the amount of Pd(OAc)₂ was 20 mol %. As a result, a divinylbenzene compound 405 was obtained. The ¹H-NMR spectrum of the obtained compound was as shown below. 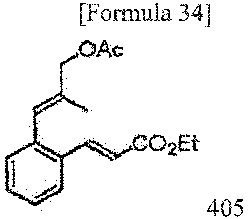 ¹H NMR (300MHz, CDCl₃) δ 1.34 (t, J = 7.14 Hz, 3 H), 1.89 (s, 3H), 2.12 (s, 3 H) 4.27 (q, J=7.11 Hz, 2 H), 4.63 (s, 2 H), 5.70 (d, J = 12.3 Hz, 1 H), 6.52 (s, 1 H), 7.30-7.50 (m, 4 H), 8.29 (d, J = 12.6 Hz, 1 H).

### [Examples 2-9 to 2-13]

Reactions were performed in the same manner as in Example 2-1 except that the solvents shown in Table 2 were substituted for acetic acid. As a result, divinylbenzene compounds were obtained in minute yields. In these examples, the yields were not calculated.

**[Table 2]**

| | | R₁ | R₁ | Catalyst (mol %) | Solvent | Yield (%) |
|---|---|---|---|---|---|---|
| Example 2 | 1 | methyl group | H | 10 | acetic acid | 17 |
| | 2 | methyl group | H | 20 | acetic acid | 24 |
| | 3 | methyl group | H | 30 | acetic acid | 14 |
| | 4 | phenyl group | H | 20 | acetic acid | 15 |
| | 5 | t-butyl group | H | 20 | acetic acid | 9 |
| | 6 | -CH₂OC(=O)CH₃ group | H | 20 | acetic acid | 8 |
| | 7 | TIPS* | H | 20 | acetic acid | 11 |
| | 8 | methyl group | methyl group | 20 | acetic acid | 10 |
| | 9 | methyl group | H | 20 | DMF | ** |
| | 10 | methyl group | H | 20 | 1,4-dioxane | ** |
| | 11 | methyl group | H | 20 | TFA | ** |
| | 12 | methyl group | H | 20 | acetic acid/DMF=1:1 | ** |
| | 13 | methyl group | H | 20 | acetonitrile/acetic acid=1:1 | ** |

| | | | | | | |
|---|---|---|---|---|---|---|
| * Triisopropylsilyl group ** Not calculated | | | | | | |

### [Comparative Examples 2-1 and 2-2]

Reactions were performed in the same manner as in Example 2-1 except that PdCl₂ and Pd(OTs)₂(NCMe)₂, respectively, were substituted for Pd(OAc)₂. However, no divinylbenzene compounds were obtained.

### [Comparative Example 2-3]

A reaction was performed in the same manner as in Example 2-4 except that the compound used in Comparative Example 1-1 was substituted for a vinylbenzene compound. However, any reaction of substitution with the ethyl acrylate-derived group at the ortho position did not occur.

### [Comparative Example 2-4]

A reaction was performed in the same manner as in Example 2-4 except that the compound used in Comparative Example 1-2 was substituted for a vinylbenzene compound. However, any reaction of substitution with the ethyl acrylate-derived group at the ortho position did not occur.

### [Example 3]

The reaction scheme of this example is shown below.

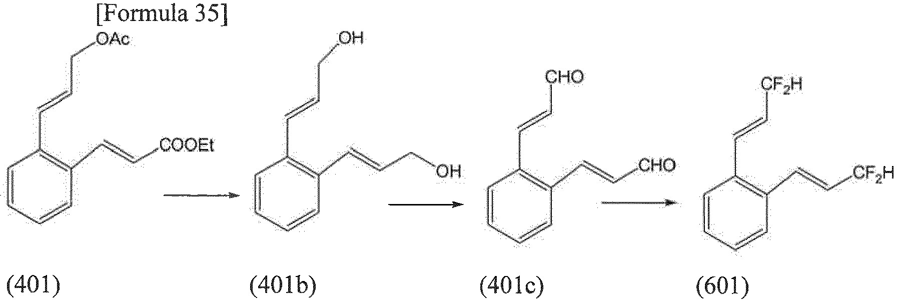

The vinylbenzene compound 401 was obtained in the same manner as in Example 2-2. Subsequently, 24.8 mg (0.09 mmol) of the vinylbenzene compound and 1 mL of methylene chloride were charged in a flask, the mixture was stirred to prepare a solution, and the temperature of the content was lowered to -78°C. Into the flask, 0.46 mL of a 1.04M DIBAL (diisobutyl aluminum hydride) solution in hexane (DIBAL: 0.45 mmol) was added dropwise under an argon atmosphere. After a reduction reaction was performed at -78°C for 3 hours, the reaction was quenched with an aqueous solution of Rochelle salt (potassium sodium tartrate). Subsequently, an organic phase was separated from the mixture in the flask. The remaining aqueous phase was extracted with methylene chloride three times to collect an organic phase. The previously separated organic phase and the organic phase obtained by the extraction with methylene chloride were combined, and the combined organic phase was washed with a saturated aqueous solution of NaCl and then dried over MgSO₄. Further, the organic phase was placed under reduced pressure to remove the organic solvent. The resulting crude product was purified by silica gel chromatography to give a compound 401b. The yield was 95%. The ¹H-NMR spectrum was as shown below.

### [Formula 36]

¹H NMR (300MHz, CDCl₃) δ 4.34-4.37 (m, 2 H), 6.24 (dt, J = 15.9, 5.70 Hz, 2 H), 6.92 (d, J = 15.6 Hz, 2 H), 7.27-7.45 (m, 4 H)

In a flask, 7.2 mg (0.04 mmol) of the compound 401b and 1 mL of methylene chloride were charged and the mixture was stirred to prepare a solution. The temperature of the content was adjusted to room temperature, 70 mg of MnO₂ (0.8 mmol) was charged in the flask under an argon atmosphere, and an oxidation reaction was performed for 12 hours. After that, the solvent was removed under reduced pressure. The resulting product was purified by silica gel chromatography to give a compound represented by chemical formula 401c that has aldehyde groups. The yield was 98%. The ¹H-NMR spectrum was as shown below.

### [Formula 37]

¹H NMR (300MHz, CDCl₃) δ 6.69 (dd, J = 15.6, 8.1 Hz, 2 H) 7.50-7.68 (m, 4 H), 7.88 (d, J = 15.9 Hz, 2 H), 9.80 (d, J = 7.5 Hz, 2 H)

In 5 mL of methylene chloride, 190 mg (1 mmol) of the compound 401c was dissolved to prepare a solution and the solution was charged in a PTFE tube. Subsequently, under an argon atmosphere, 851 mg (3.4 mmol) of FLOULEAD (Ube Industries, Ltd.), which is a fluorinating agent, and 4.6 µL (0.1 mmol) of ethanol were added into the PTFE tube and a fluorination reaction was performed at room temperature for 12 hours. After that, an aqueous solution of Na₂CO₃ was added into the tube and the mixture was stirred for 3 hours. Next, an organic phase was separated from the reaction mixture. The remaining aqueous phase was extracted with diethyl ether three times to collect an organic phase. The previously separated organic phase and the organic phase obtained by the extraction with diethyl ether were combined, and the combined organic phase was washed with a saturated aqueous solution of NaCl and then dried over MgSO₄. Further, the organic phase was placed under reduced pressure to remove the organic solvent. The resulting crude product was purified by silica gel chromatography to give a compound 601. The yield was 53%. With regard to the obtained compound, the ¹H-NMR spectrum and ¹⁹F-NMR (measured with AV300M from Bruker at 300 MHz using CDCl₃ and BTF (benzotrifluoride) as an internal standard) were as shown below.

### [Formula 38]

¹H NMR (300MHz, CDCl₃) δ 6.23-6.09 (m, 2 H), 6.28 (td, J = 55.5, 5.40 Hz, 2 H), 7.16 (dt, J = 16.2, 3.60 Hz 2 H), 7.36-7.51 (m, 4 H).
¹⁹F NMR (282 MHz, CDCl₃) δ -110.47 (dd, J = 58.1, 6.49 Hz, 4F)

### [Comparative Example 3]

In acetic acid (Kanto Chemical Co., Inc.), 4.5 mg of Pd(OAc)₂ (a product of Sigma-Aldrich) and 2.0 mmol of Cu(OAc)₂ as an oxidizing agent were dissolved to prepare an acetic acid solution.

In a flask, 1.0 mmol of a vinylbenzene compound represented by the chemical formula 100' wherein R₁ is a methyl group and R₂ is a hydrogen atom and 33 µL (3.0 mmol) of ethyl acrylate were charged. Subsequently, the acetic acid solution was charged in the flask at room temperature under an argon atmosphere. This step was performed such that the amount of Pd(OAc)₂ was 10 mol % relative to the vinylbenzene compound. The flask was heated to adjust the internal temperature to 100°C and the mixture was stirred for 12 hours to perform a reaction. After that, the reaction mixture was cooled and a saturated aqueous solution of NaCO₃ was charged in the flask to stop the reaction. An organic phase was separated from the mixture in the flask. The remaining aqueous phase was extracted with ether three times to collect an organic phase. The previously separated organic phase and the organic phase obtained by the extraction with ether were combined, and the combined organic phase was washed with a saturated aqueous solution of NaCl and then dried over MgSO₄. Further, the organic phase was placed under reduced pressure to remove the organic solvent. The resulting crude product was purified by silica gel chromatography. However, no divinylbenzene compound represented by chemical formula 400' was obtained.

In addition, reactions were tried using Pd(OTs)₂(NCMe)₂ as a catalyst, Cu(OAc)₂, Ag₂O₃, or free oxygen (1 atm) as an oxidizing agent, or THF or DMSO as a solvent, but no divinylbenzene compound was obtained.

In this example, it had been expected that a catalyst cycle as shown in the scheme below would occur, but it was revealed that the cycle actually does not occur.

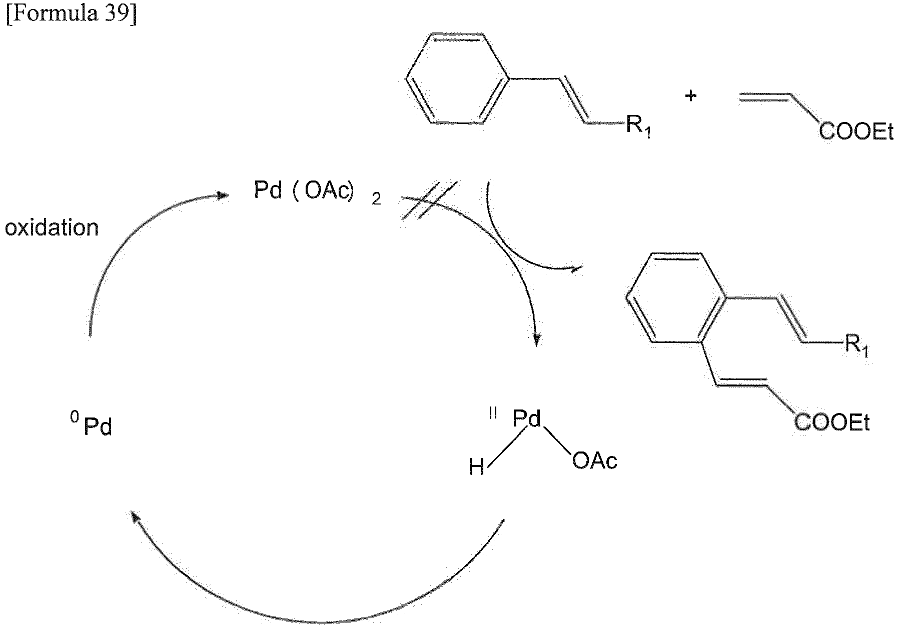

## Claims

1. A method for producing a polyvalent vinyl aromatic compound comprising:
a step of allowing (1) a vinyl aromatic compound represented by the following chemical formula 1: 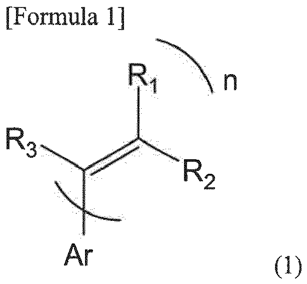
wherein Ar is a monocyclic aromatic hydrocarbon group or a ring-cumulated hydrocarbon group formed by linkage of a plurality of the aromatic hydrocarbon groups via a single bond or an unsaturated bond, has a vinyl group on a carbon atom, and may have a substituent on a carbon atom that is not adjacent to the carbon atom having the vinyl group,
R₁ is a group represented by -CH₂OC(=O)R₄ (wherein R₄ is an alkyl group having 1 to 20 carbon atoms), an alkyl group having 1 to 20 carbon atoms, either a monocyclic aromatic hydrocarbon group which may have a substituent or a ring-cumulated hydrocarbon group which may have a substituent and which is formed by linkage of a plurality of the aromatic hydrocarbon groups via a single bond or an unsaturated bond, a trialkylsilyl group, or a hydrogen atom,
R₂ is a hydrogen atom or an alkyl group having 1 to 5 carbon atoms,
R₃ is a hydrogen atom or an alkyl group having 1 to 20 carbon atoms, and
n is an integer of 1 to m, provided that when Ar is a monocyclic aromatic hydrocarbon group, m is a number obtained by subtracting 1 from the number of carbon atoms that constitute the aromatic ring represented by Ar or, when Ar is a ring-cumulated hydrocarbon group, m is a number obtained by subtracting 1 from the number of carbon atoms that are not involved in a single bond or an unsaturated bond among the carbon atoms that constitute the aromatic ring,
to react with
(2) a vinyl compound represented by the following chemical formula 2: 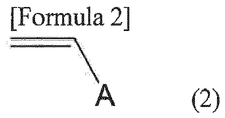
wherein A is R₅ or a group represented by -COOR₅, where R₅ is an alkyl group having 1 to 20 carbon atoms or a halogenated alkyl group having 1 to 20 carbon atoms,
in the presence of:
(3-1) a metal complex represented by the following chemical formula 3 and (3-2) a compound in the class of percarboxylic acids or a hypervalent iodine compound, 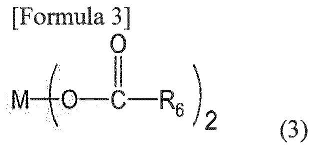
wherein M is Pd, Pt, Au, Rh, or Ir and R₆ is an alkyl group having 1 to 5 carbon atoms, a phenyl group, or a phenyl group having, as a substituent, an alkyl group having 1 to 5 carbon atoms,
to introduce a vinyl group derived from the vinyl compound onto a carbon atom which has no substituent and which is adjacent to the carbon atom to which the vinyl group of the vinyl aromatic compound is attached,
the polyvalent vinyl aromatic compound being represented by the following chemical formula 4: 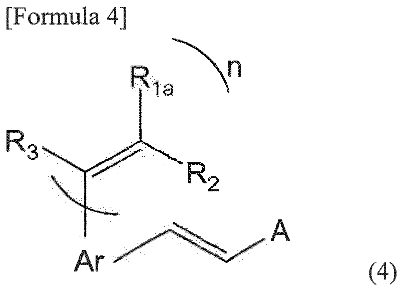
wherein Ar and n are defined as stated above,
R₁ₐ is R₁ or, when R₁ in the chemical formula 1 is a methyl group, R₁ₐ is -CH₂OC(=O)R₆, wherein R₁ and R₆ are defmed as stated above, and
R₂, R₃, and A are defined as stated above.

2. The method according to Claim 1, wherein M is Pd and R₆ is a methyl group or a phenyl group, in the metal complex of the chemical formula 3.

3. The method according to Claim 1 or 2, wherein the compound in the class of percarboxylic acids is a perbenzoate ester and the hypervalent iodine compound is diacyloxyiodobenzene.

4. The method according to any one of Claims 1 to 3, wherein, relative to 1 mol of the vinyl aromatic compound of the chemical formula 1, the vinyl compound of the chemical formula 2 is used in an amount of 2 to 5 mol, the metal complex of the chemical formula 3 is used in an amount of 0.1 to 0.5 mol, and the compound in the class of percarboxylic acids or the hypervalent iodine compound is used in an amount of 1 to 3 mol.

5. The method according to any one of Claims 1 to 4, wherein, in the vinyl aromatic compound of the chemical formula 1, Ar is a phenyl group, R₁ is a phenyl group, an alkyl group having 1 to 4 carbon atoms, a silyl group, or a group represented by -CH₂OC(=O)R₄ where R₄ is an alkyl group having 1 to 20 carbon atoms, and R₂ and R₃ are independently a hydrogen atom or a methyl group and wherein, in the vinyl compound of the chemical formula 2, A is a group represented by -COOR₅ where R₅ is an alkyl group having 1 to 20 carbon atoms.

6. The method according to any one of Claims 1 to 5, wherein said reaction is performed in a carboxylic acid solvent.

7. A method for producing a polyvalent vinyl aromatic compound comprising:
a step of producing a polyvalent vinyl aromatic compound by allowing a vinyl aromatic compound represented by the following chemical formula 1a: 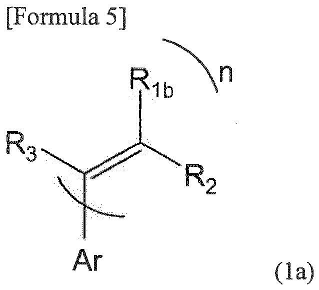
wherein Ar and n are defined as stated above,
R_{1b} is a methyl group or -CH₂OC(=O)R₄ wherein R₄ is an alkyl group having 1 to 20 carbon atoms,
R₂ is a hydrogen atom or an alkyl group having 1 to 5 carbon atoms, and
R₃ is a hydrogen atom or an alkyl group having 1 to 20 carbon atoms,
to react with
(2) a vinyl compound represented by the following chemical formula 2a: 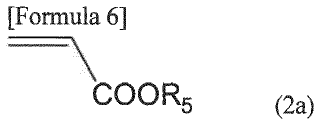
wherein R₅ is an alkyl group having 1 to 20 carbon atoms or a halogenated alkyl group having 1 to 20 carbon atoms,
in the presence of:
(3-1) a metal complex represented by chemical formula 3 and (3-2) a percarboxylic acid or a hypervalent iodine compound,
to introduce a vinyl group derived from the vinyl compound onto a carbon atom which has no substituent and which is adjacent to a carbon atom to which a vinyl group is attached in the vinyl aromatic compound,
the second mentioned polyvalent vinyl aromatic compound being represented by the following chemical formula 4a: 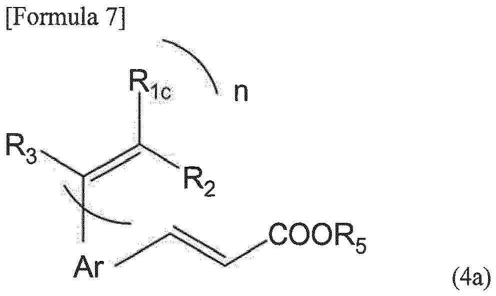
wherein Ar and n are defined as stated above and R_{1c} is -CH₂OC(=O)R₄ or, when R_{1b} of the vinylbenzene compound of the chemical formula 1a is a methyl group, R_{1c} is -CH₂OC(=O)R₆;
a step of reducing an ester group of the compound obtained by the introduction step to a hydroxy group;
a step of oxidizing the hydroxy group of a compound obtained by the reduction step to an aldehyde group; and
a step of converting the aldehyde group into a difluoromethyl group by reacting a compound obtained by the oxidation step and a compound represented by the following chemical formula 5: 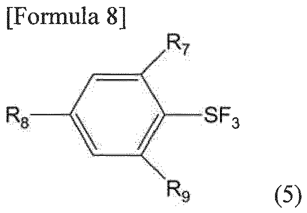
wherein R₇ and R₉ are independently a hydrogen atom or an alkyl group having 1 to 8 carbon atoms and R₈ is a hydrogen atom, a halogen atom, or an alkyl group having 1 to 8 carbon atoms, and
the first mentioned polyvalent vinyl aromatic compound being represented by the following chemical formula 6: 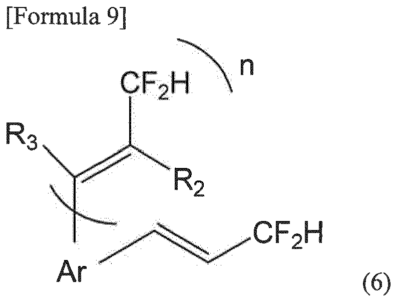 wherein Ar, R₂, R₃, and n are defined as stated above.

8. A polyvalent vinyl aromatic compound represented by the following chemical formula 4: 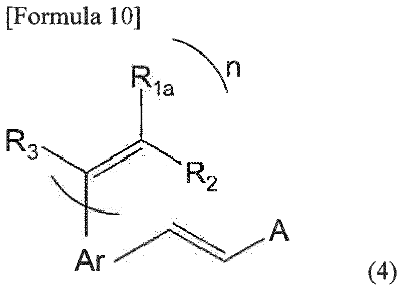 wherein Ar, R₁ₐ to R₃, A, and n are defined as stated above and the -CH₂=CH₂-A group is attached to a carbon atom that is adjacent to a carbon atom to which the -CHR₃=CR₁ₐR₂ group is attached.

9. A polyvalent vinyl aromatic compound represented by the following chemical formula 4': 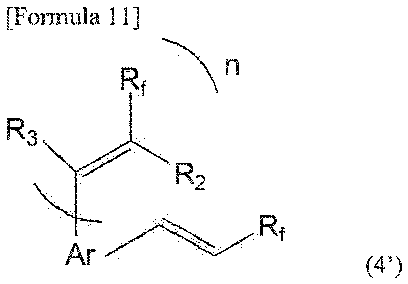 wherein Ar is defined as stated above, R_{f} are independently a difluoromethyl group or a perfluoromethyl group, R₂, R₃, and n are defined as stated above, and the -CH₂=CH₂-R_{f} group is attached to a carbon atom that is adjacent to a carbon atom to which the -CHR₃=CR_{f}R₂ group is attached.
